# FASCICULE DE BREVET EUROPEEN

(11) **EP 2 498 680 B1**
(45) Date de publication et mention de la délivrance du brevet: **16.10.2013**
(21) Numéro de dépôt: 10779707.8
(22) Date de dépôt: 10.11.2010
(51) Int. Cl.: A61B 5/1455

(54) **DISPOSITIF DE MESURE D'UNE ACTIVITE DE LA MOELLE EPINIERE D'UN VERTEBRE**
VORRICHTUNG ZUR MESSUNG DER RÜCKENMARKSAKTIVITÄT BEI EINEM WIRBEL
DEVICE FOR MEASURING THE ACTIVITY OF THE SPINAL CORD OF A VERTEBRATE

(30) Priorité: 13.11.2009 FR 0905481
(43) Date de publication de la demande: 19.09.2012
(73) Titulaire: Université Pierre et Marie Curie - Paris 6, 75005 Paris (FR); Polyvalor, Limited Partnership, Montréal, QC H3V 1H8 (CA); Valorisation-Recherche, Limited Partnership, Montréal, QC H3V 1H8 (CA)
(72) Inventeur: GOGUIN, Alexandre, F-75015 Paris (FR); LESAGE, Frédéric, H2V 1 P1 Outremont (Québec) (CA); ROSSIGNOL, Serge, H4A 2J9 Montréal (CA); BENALI, Habib, F-91700 Sainte-Genevieve des Bois (FR)
(74) Mandataire: Parzy, Benjamin Alain
(86) Numéro de dépôt international: PCT/EP2010/006832
(87) Numéro de publication internationale: WO 2011/057765

(56) Documents cités:
- WO-A2-2007/098385
- US-A1- 2006 036 324
- US-A1- 2006 224 088
- US-A1- 2006 247 773
- MACNAB ANDREW J MD FRCPC ET AL: "Near Infrared Spectroscopy for Intraoperative Monitoring of the Spinal Cord", SPINE, LIPPINCOTT WILLIAMS & WILKINS, US, vol. 27, 1 janvier 2002 (2002-01-01), pages 17-20, XP009135829, ISSN: 0362-2436
- SIMONOVICH MARYANA MSC ET AL: "Real-Time Monitoring of Mitochondrial NADH and Microcirculatory Blood Flow in the Spinal Cord", SPINE, LIPPINCOTT WILLIAMS & WILKINS, US, vol. 33, 1 janvier 2008 (2008-01-01), pages 2495-2502, XP009135828, ISSN: 0362-2436

## Description

L'invention concerne un dispositif de mesure d'une activité de la moelle épinière d'un vertébré.

Par activité on entend ici toute variation d'une propriété optique, chimique ou physique de la moelle épinière pouvant caractériser notamment une réaction neuronale à un acte moteur ou cognitif.

### ARRIERE PLAN DE L'INVENTION

Un acte moteur résulte d'un ensemble de processus énergétiques acheminant un signal nerveux via la moelle épinière jusqu'aux organes effecteurs. Or une lésion partielle ou totale de la moelle épinière entraine une dégénérescence des voies nerveuses qui ne peuvent plus transmettre de signaux aux neurones spinaux sous-jacents à la lésion, ce qui peut conduire à une paralysie ainsi qu'à une interruption des voies ascendantes vers le cerveau qui mène à une abolition des sensations.

La compréhension des mécanismes d'acheminement du signal nerveux le long de la colonne vertébrale est nécessaire pour mettre au point des traitements thérapeutiques les plus efficaces possibles.

Il est connu qu'une variabilité dans la répartition en hémoglobine oxygénée (HbO) et désoxygénée (Hb) est observable près des vaisseaux et capillaires irriguant les régions neuronales du cortex et de la moelle épinière lors d'une activité cognitive ou motrice.

En se basant sur un dispositif d'imagerie optique permettant la détection de ces variations de volume et d'oxygénation sanguine, les tissus neuronaux de l'encéphale ont ainsi pu être étudiés. Cependant la technologie employée est de taille trop importante pour être placée sur une autre partie du corps ou pour être implantée de façon chronique chez un vertébré. Alors qu'une trépanation suffit pour accéder aux tissus corticaux, l'accès aux tissus médullaires est plus délicat et nécessite une laminectomie. Enfin, le dispositif d'imagerie optique connu comporte majoritairement des fibres optiques dont l'utilisation dans la moelle épinière est délicate car leur géométrie rend difficile l'intégration des fibres de façon simple afin de mesurer la réponse hémodynamique.

Un tel dispositif de mesure intraopérative de l'activité de la moelle épinière d'un vertébré est notamment connu du document « Near Infrared Spectroscopy for Intraoperative Monitoring of Spinal Cord », AJ Macnab et al, Lippincott Williams and Wilkins, US, vol. 27. pages 17-20.

Actuellement, le principal moyen d'étude de la moelle épinière dans son ensemble reste donc l'Imagerie par Résonance Magnétique (IRM) qui permet une visualisation de la moelle et une analyse des tissus médullaires. L'étude restant cependant externe, l'activité neuronale perçue dans les tissus médullaires est brouillée et difficilement interprétable d'un patient à un autre.

### OBJET DE L'INVENTION

L'invention a pour objet un dispositif implantable, permettant de mesurer une activité de la moelle épinière d'un vertébré tout en s'affranchissant des difficultés précitées.

### BREVE DESCRIPTION DE L'INVENTION

En vue de la réalisation de ce but, on propose selon l'invention un dispositif de mesure d'une activité de la moelle épinière d'un vertébré comportant au moins une sonde principale implantable de façon chronique, la sonde principale étant conformée pour être fixée à une apophyse épineuse d'une vertèbre et maintenir en position de part et d'autre de la vertèbre au moins un émetteur pour émettre une onde susceptible d'interagir avec la moelle épinière et au moins un récepteur associé pour recevoir l'onde ayant interagi avec la moelle épinière et générer un signal représentatif de l'activité de la moelle épinière.

La sonde principale se place très simplement en incisant à l'endroit de la vertèbre concernée et en écartant les muscles de part et d'autre de cette vertèbre de sorte à chevaucher celle-ci. Il reste alors à fixer la sonde principale à la vertèbre en vissant ou en collant celle-ci à l'apophyse. L'intervention est simple et rapide.

### BREVE DESCRIPTION DES DESSINS

L'invention sera mieux comprise à la lumière de la description qui suit en référence aux figures des dessins annexés parmi lesquelles :
- la figure 1 est une vue en perspective d'une vertèbre équipée d'une sonde principale selon l'invention ;
- la figure 2 est une vue en coupe transversale de la vertèbre équipée d'une sonde principale illustrée à la figure 1 ;
- la figure 3 est une vue en perspective de la sonde principale illustrée à la figure 1 ;
- la figure 4 est une vue en coupe transversale d'une vertèbre équipée d'une sonde principale et d'une sonde auxiliaire associée, selon l'invention ;
- la figure 5 est une vue en perspective de la sonde auxiliaire illustrée à la figure 4.

### DESCRIPTION DETAILLEE DE L'INVENTION

L'invention est illustrée en application à une vertèbre de chat. Cette application n'est bien sûr pas limitative.

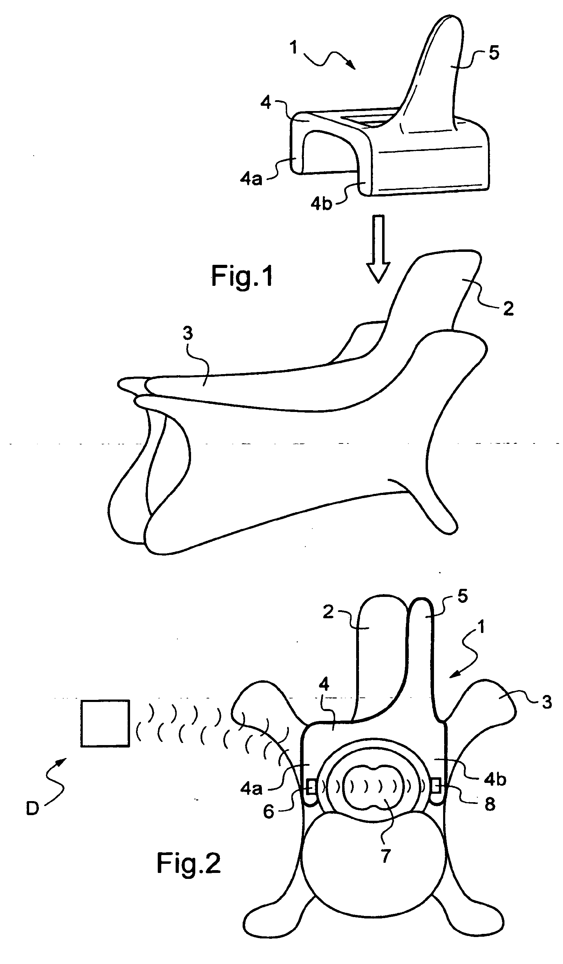

En référence aux figures 1 à 3, le dispositif de mesure de l'invention comporte une sonde principale 1 conformée pour être fixée sur une apophyse 2 d'une vertèbre 3. La sonde principale 1, en résine transparente aux infrarouges, se présente sous la forme d'un cavalier 4 comportant deux ailes 4a et 4b et d'une palette 5 qui s'étend en saillie du cavalier 4.

Cette morphologie permet une intervention chirurgicale simplifiée, le chirurgien n'ayant qu'à écarter les muscles situés sur les côtés de la vertèbre 3 et à poser le cavalier 4 sur l'apophyse 2 selon la direction de la flèche visible à la figure 1. Dans cette position, le cavalier 4 chevauche la partie de la vertèbre formant le canal médullaire et la palette 5 s'étend en regard de l'apophyse 2. La sonde principale 1 est alors fixée préférentiellement au niveau de la palette 5 à l'apophyse 2, par exemple à l'aide d'une colle biocompatible, ou encore à l'aide de vis en titane.

L'aile 4a porte un émetteur infrarouge 6 dont le rayonnement traverse la moelle épinière 7 de la vertèbre et est capté, après son interaction avec les tissus médullaires, par un récepteur infrarouge 8 porté par l'autre aile 4b. Le rayonnement infrarouge traverse la paroi osseuse et interagit avec l'hémoglobine du sang contenu dans la moelle 7, ce qui permet de suivre une activité vasculaire de la moelle 7 lorsqu'elle est sollicitée. Le récepteur 8 transforme le rayonnement reçu en un signal électrique représentatif de cette activité vasculaire, elle-même représentative d'une activité neuronale.

La sonde principale 1 comporte des moyens électroniques 9 pour transformer ce signal électrique en données numériques. Ces données numériques sont ici stockées dans une mémoire et transmises ultérieurement. A cet effet, la sonde principale 1 comporte un émetteur infrarouge 10 et un récepteur infrarouge 11 pour communiquer par trame binaire avec un émetteur-récepteur infrarouge distant de la sonde principale 1. Alternativement, les données numériques sont transmises en temps réel, sans être stockées.

Les données numériques ainsi générées sont avantageusement envoyées vers un récepteur-émetteur distant D pour suivre l'activité vasculaire de la moelle épinière en réponse à une stimulation de celle-ci. L'émetteur-récepteur peut par exemple être associé à un boîtier de contrôle externe adapté à commander des actionneurs agissant sur l'organisme du vertébré pour pallier une insuffisance de celui-ci. Par exemple, le boîtier de contrôle peut être utilisé pour commander une pompe ou un implant délivrant une substance chimique en réponse à la détection d'une activité de la moelle au moyen de la sonde principale.

L'alimentation électrique de la sonde principale 1 est réalisée ici à l'aide d'un circuit extérieur à la sonde principale 1 qui fournit un champ magnétique variable entrainant ainsi l'apparition d'un courant dans une antenne 12 disposée ici dans la palette 5. Les moyens électroniques 9 sont adaptés à conditionner le potentiel induit dans l'antenne 12 en un potentiel utilisable directement par la sonde principale 1. Ici, afin de permettre à la sonde de fonctionner même en étant éloignée de la source inductive, on utilise de préférence ce potentiel pour charger une batterie 13 embarquée.

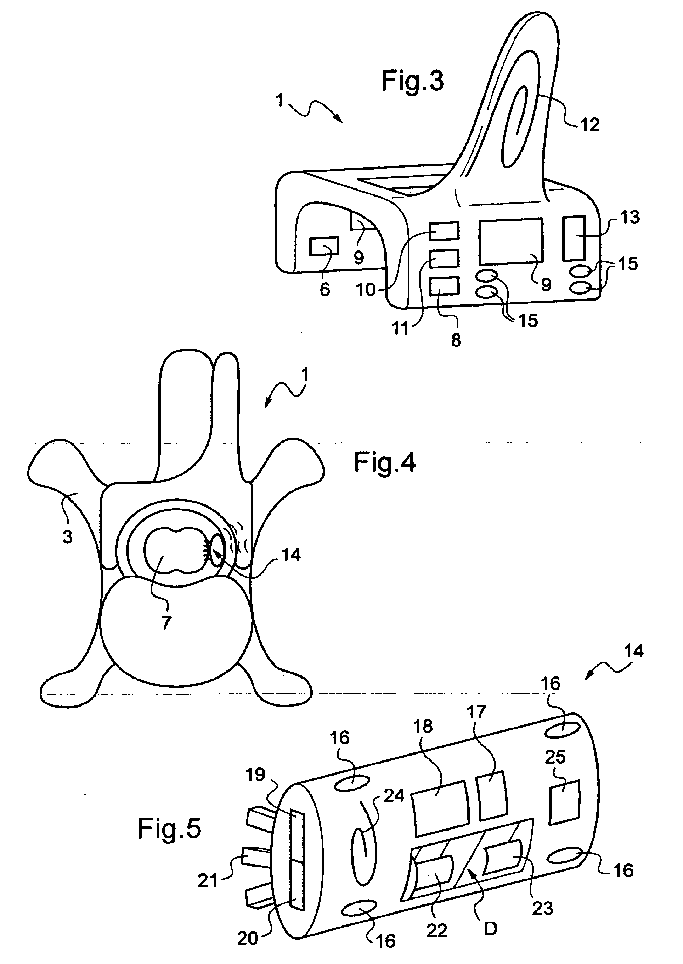

En référence aux figures 4 et 5, et selon un aspect particulier de l'invention, l'émetteur-récepteur distant D peut être associé à une sonde auxiliaire 14 implantée à l'intérieur de la vertèbre 3, pour être en contact direct avec la moelle épinière 7. Selon un mode d'implantation privilégié, une fois les muscles entourant la vertèbre écartés, le chirurgien pratique une laminectomie de la vertèbre, introduit la sonde auxiliaire 14 dans le canal médullaire et reconstitue la vertèbre à l'aide d'une résine polymère. En référence aux figures 3,4 et 5, et selon un mode de stabilisation privilégié, la stabilisation en position de la sonde auxiliaire 14 est assurée par liaison magnétique avec la sonde principale 1 : chaque sonde comporte des aimants 15,16, les aimants d'une même sonde étant d'une même polarité, opposée à celle des aimants de l'autre sonde. L'épaisseur de la vertèbre 3 est suffisamment faible pour que les aimants 15,16 s'attirent à travers la paroi osseuse et ainsi stabilisent la sonde auxiliaire 14 relativement à la sonde principale 1. Cette fixation est avantageuse par rapport à une fixation à l'aide de fils chirurgicaux en ce qu'elle simplifie l'opération chirurgicale.

La sonde auxiliaire 14 peut remplir plusieurs fonctions :
- mesurer une activité neuronale de la moelle épinière 7. A cet effet, la sonde auxiliaire 14 comporte un capteur 17 réagissant à la présence de certaines molécules biologiques dans le liquide céphalo-rachidien en produisant un signal électrique. La sonde auxiliaire 14 comporte des moyens électroniques 18 pour transformer ce signal en données numériques.
- stimuler l'activité neuronale de la moelle épinière par des moyens chimique et/ou électrique. A cet effet, la sonde auxiliaire 14 comporte un réservoir 19, muni d'une membrane, pour stocker des composés chimiques ; un micromoteur 20 coopère avec la membrane pour déplacer sélectivement celle-ci, ce qui provoque l'expulsion des composés chimiques dans la moelle épinière 7. En outre, des électrodes 21 peuvent imposer un potentiel local aux axones de la moelle épinière 7 en contact avec les électrodes 21.

L'émetteur-récepteur D de la sonde auxiliaire 14 comporte ici un émetteur infrarouge 22 et un récepteur -infrarouge 23 qui communiquent par trame binaire avec l'émetteur infrarouge 10 et le récepteur infrarouge 11 de la sonde principale 1. Ainsi, aucune liaison filaire n'est nécessaire entre les deux sondes pour transmettre les données générées par les moyens électroniques 18 ou pour recevoir des ordres de la sonde principale 1. De même, l'alimentation de la sonde auxiliaire 14 est réalisée sans fil : selon un dispositif privilégié, une antenne 24 est en liaison par induction avec l'antenne 12 de la sonde principale 1. Les moyens électroniques 18 sont adaptés à conditionner le potentiel induit dans l'antenne 24 en un potentiel utilisable directement par la sonde auxiliaire 14. Ici on utilise de préférence ce potentiel pour charger une batterie 25 embarquée.

On remarquera que la sonde auxiliaire doit être miniaturisée du fait de son implantation au plus près de la moelle épinière, organe hypersensible. De par sa taille, la sonde auxiliaire est limitée en autonomie et nécessite d'être reliée à une sonde de taille plus importante, ici la sonde principale, pour être alimentée régulièrement.

L'invention n'est pas limitée à ce qui vient d'être décrit et englobe toute variante entrant dans le cadre défini par les revendications.

En particulier, bien que l'on ait indiqué que la communication entre la sonde auxiliaire- 14---et la sonde principale 1, ainsi qu'entre la sonde principale 1 et un émetteur-récepteur distant de la sonde principale 1 se faisait par rayonnement infrarouge, on pourra utiliser tout autre mode de communication comme la communication RFID ou bien encore, dans le cas de la sonde principale 1 et de l'émetteur-récepteur distant, une communication avec fils. En outre, bien qu'on ait indiqué que la sonde principale 1 comportait un même système de communication pour communiquer avec la sonde auxiliaire 14 et avec l'émetteur-récepteur distant de la sonde principale, on pourra bien évidemment proposer une sonde possédant deux systèmes de communication distincts.

Bien que l'on ait décrit dans la sonde principale 1 un système de mesure d'une activité de la moelle épinière 7 par émission d'ondes électromagnétiques dans l'infrarouge, on pourra employer d'autres techniques connues comme l'émission d'ondes ultrasonores.

Bien que l'on ait décrit que la sonde auxiliaire était implantée à l'intérieur de la vertèbre 3 pour être en contact direct avec la moelle épinière 7, on pourra plus généralement implanter la sonde auxiliaire au contact de tout tissu fonctionnel.

En variante, il est envisageable de coupler la sonde auxiliaire à une pompe osmotique de type Alzet.

## Revendications

1. Dispositif de mesure d'une activité de la moelle épinière d'un vertébré, **caractérisé en ce que** le dispositif comporte au moins une sonde principale (1) conformée pour être fixée à une apophyse épineuse (2) d'une vertèbre (3) et maintenir en position de part et d'autre de la vertèbre au moins un émetteur (6) pour émettre une onde susceptible d'interagir avec la moelle épinière (7) et au moins un récepteur associé (8) pour recevoir l'onde ayant interagi avec la moelle épinière et générer un signal représentatif de l'activité de la moelle épinière.

2. Dispositif de mesure selon la revendication 1, dans lequel la sonde principale est conformée en cavalier (4) pour chevaucher la vertèbre, et comporte une palette (5) qui s'étend pour- -être en regard de l'apophyse lorsque la sonde principale est en position sur la vertèbre.

3. Dispositif de mesure selon la revendication 1, dans lequel l'onde émise par l'émetteur (6) de la sonde principale et reçue par le récepteur (8) associé est un rayonnement infrarouge.

4. Dispositif de mesure selon la revendication 1, dans lequel la sonde principale comporte des moyens de communication (10,11) pour communiquer à distance avec au moins un émetteur-récepteur distant (D), les moyens de communication comportant au moins un émetteur infrarouge (10) émettant des signaux vers l'émetteur-récepteur distant (D) et au moins un récepteur infrarouge (11) recevant des signaux de l'émetteur-récepteur distant (D).

5. Dispositif de mesure selon la revendication 4, dans lequel l'émetteur-récepteur distant (D) est associé à au moins une sonde auxiliaire (14) placée en contact direct avec un tissu fonctionnel, notamment la moelle épinière, ladite sonde comportant des moyens de stimulation (19, 20, 21) du tissu fonctionnel et/ou des moyens de mesure (17) d'une activité du tissu fonctionnel.

6. Dispositif de mesure selon la revendication 5, dans lequel la sonde auxiliaire est prévue pour être placée en contact avec la moelle épinière, la sonde auxiliaire et la sonde principale comportant des moyens de stabilisation magnétique (15,16) de la sonde auxiliaire en position dans la vertèbre.

7. Dispositif de mesure selon la revendication 5, dans lequel les moyens de stimulation de la moelle épinière comportent des électrodes (21) pour imposer un potentiel local à des axones en contact avec les électrodes.

8. Dispositif de mesure selon la revendication 5, dans lequel les moyens de stimulation de la moelle épinière comportent un réservoir (19) qui stocke des molécules et qui est associé à des moyens (20) de libération progressive desdites molécules dans la moelle épinière.

9. Dispositif de mesure selon la revendication 5, dans lequel la sonde auxiliaire comporte des moyens inductifs pour recevoir une énergie électrique à distance, les moyens inductifs comportant au moins une antenne (24)

10. Dispositif de mesure selon la revendication 1, dans lequel la sonde principale comporte des moyens inductifs pour recevoir une énergie électrique à distance, les moyens inductifs comportant au moins une antenne (12).

## Patentansprüche

1. Vorrichtung zur Messung der Rückenmarksaktivität eines Wirbels, **dadurch gekennzeichnet, dass** die Vorrichtung mindestens eine Hauptsonde (1) umfasst, die so ausgebildet ist, dass sie an einem Dornfortsatz (2) eines Wirbels (3) fixiert werden kann und auf beiden Seiten des Wirbels mindestens einen Sender (6) in Position halten kann, um eine Welle auszusenden, die in der Lage ist, mit dem Rückenmark (7) zu interagieren, und mindestens einen damit verbundenen Empfänger (8) umfasst, um die Welle zu empfangen, die mit dem Rückenmark interagiert hat, und ein Signal zu erzeugen, das repräsentativ für die Aktivität des Rückenmarks ist.

2. Messvorrichtung nach Anspruch 1, bei der die Hauptsonde als Reiter ausgebildet ist (4), damit sie auf den Wirbel aufgesetzt werden kann, und eine Fahne (5) umfasst, die sich so erstreckt, dass sie sich gegenüber der Apophyse befindet, wenn die Hauptsonde auf dem Wirbel positioniert ist.

3. Messvorrichtung nach Anspruch 1, bei der die durch den Sender (6) der Hauptsonde ausgesandte und durch den damit verbundenen Empfänger (8) empfangene Welle eine Infrarotstrahlung ist.

4. Messvorrichtung nach Anspruch 1, bei der die Hauptsonde Kommunikationsmittel (10, 11) zur Fernkommunikation mit mindestens einem entfernten Sender-Empfänger (D) umfasst, wobei die Kommunikationsmittel mindestens einen Infrarotsender (10), der Signale in Richtung des entfernten Sender-Empfängers (D) aussendet, und mindestens einen Infrarotempfänger (11), der Signale des entfernten Sender-Empfängers (D) empfängt, umfassen.

5. Messvorrichtung nach Anspruch 4, bei der der entfernte Sender-Empfänger (D) mit mindestens einer Hilfssonde (14) verbunden ist, die in direktem Kontakt mit einem funktionellen Gewebe, insbesondere dem Rückenmark, platziert wird, wobei die besagte Sonde Stimulationsmittel (19, 20, 21) zur Stimulation des funktionellen Gewebes und/oder Messmittel (17) zur Messung einer Aktivität des funktionellen Gewebes umfasst.

6. Messvorrichtung nach Anspruch 5, bei der die Hilfssonde (14) dazu gedacht ist, in Kontakt mit dem Rückenmark platziert zu werden, wobei die Hilfssonde und die Hauptsonde Mittel zur magnetischen Stabilisierung (15, 16) zur magnetischen Stabilisierung der Hilfssonde in ihrer Position im Wirbel umfassen.

7. Messvorrichtung nach Anspruch 5, bei der die Mittel zur Rückenmarksstimulation Elektroden (21) umfassen, um eine lokale Spannung an den Axonen, die in Kontakt mit den Elektroden sind, anzulegen.

8. Messvorrichtung nach Anspruch 5, bei der die Mittel zur Rückenmarksstimulation ein Reservoir (19) umfassen, das Moleküle speichert und mit Mitteln (20) zur schrittweisen Freigabe der besagten Moleküle in das Rückenmark verbunden ist.

9. Messvorrichtung nach Anspruch 5, bei der die Hilfssonde induktive Mittel zur Aufnahme von elektrischer Energie aus der Distanz umfasst, wobei die induktiven Mittel mindestens eine Antenne (24) umfassen.

10. Messvorrichtung nach Anspruch 1, bei der die Hauptsonde induktive Mittel zur Aufnahme von elektrischer Energie aus der Distanz umfasst, wobei die induktiven Mittel mindestens eine Antenne (12) umfassen.

## Claims

1. A measurement device for measuring activity of the spinal cord of a vertebrate, the device being **characterized in that** it includes at least one main probe (1) shaped to be fastened to a spinous process (2) of a vertebra (3) and to hold in position on opposite sides of the vertebra at least one emitter (6) for emitting a wave capable of interacting with the spinal cord (7) and at least one associated receiver (8) for receiving the wave that has interacted with the spinal cord and for generating a signal representative of the activity of the spinal cord.

2. A measurement device according to claim 1, wherein the main probe is shaped as a fork (4) for placing astride the vertebra, and includes a handle (5) that extends facing the spinous process when the main probe is in position on the vertebra.

3. A measurement device according to claim 1, wherein the wave emitted by the emitter (6) of the main probe and received by the associated receiver (8) is infrared radiation.

4. A measurement device according to claim 1, wherein the main probe includes communications means (10, 11) for communicating remotely with at least one remote transceiver (D), the communications means including at least one infrared emitter (10) emitting signals to the remote transceiver (D) and at least one infrared receiver (11) receiving signals from the remote transceiver (D).

5. A measurement device according to claim 4, wherein the remote transceiver (D) is associated with at least one auxiliary probe (14) placed in direct contact with a functional tissue, in particular the spinal cord, said probe including means (19, 20, 21) for stimulating the functional tissue and/or means (17) for measuring activity of the functional tissue.

6. A measurement device according to claim 5, wherein the auxiliary probe is designed to be placed in contact with the spinal cord, the auxiliary probe and the main probe including magnetic means (15, 16) for stabilizing the position of the auxiliary probe in the vertebra.

7. A measurement device according to claim 5, wherein the means for stimulating the spinal cord comprise electrodes (21) for imparting a local potential to axons in contact with the electrodes.

8. A measurement device according to claim 5, wherein the means for stimulating the spinal cord comprise a tank (19) storing molecules and associated with means (20) for progressively releasing said molecules into the spinal cord.

9. A measurement device according to claim 5, wherein the auxiliary probe includes inductive means for remotely receiving electrical energy, the inductive means including at least one antenna (24).

10. A measurement device according to claim 1, wherein the main probe includes inductive means for remotely receiving electrical energy, the inductive means including at least one antenna (12).
